# EUROPEAN PATENT SPECIFICATION

(11) **EP 1 604 617 B1**
(45) Date of publication and mention of the grant of the patent: **02.11.2006**
(21) Application number: 04013496.7
(22) Date of filing: 08.06.2004
(51) Int. Cl.: A61B 17/70

(54) **Rotary device for retrieving spinal column under treatment**
Drehbare Vorrichtung zur Wiederherstellung einer Wirbelsäule in Behandlung
Dispositif rotatif pour la récupération de la colonne vertébrale en traitement

(43) Date of publication of application: 14.12.2005
(73) Proprietor: A-Spine Holding Group Corp., Road Town, Tortola (VG); A-Spine Asia Co., Ltd., Taipei (TW)
(72) Inventor: Lin, Chih-I, Taipei (TW)
(74) Representative: Grünecker, Kinkeldey, Stockmair & Schwanhäusser Anwaltssozietät

(56) References cited:
- EP-A- 0 572 790
- EP-A- 0 885 598
- WO-A-01/52758
- US-A1- 2003 100 904
- PATENT ABSTRACTS OF JAPAN vol. 2003, no. 12, 5 December 2003 (2003-12-05) -& JP 2003 290243 A (A-SPINE HOLDING GROUP CORP), 14 October 2003 (2003-10-14)

## Description

### FIELD OF THE INVENTION

The present invention relates generally to a spinal surgical care device, and more particularly to a rotary device for retrieving spine column under treatment.

### BACKGROUND OF THE INVENTION

The inventor of this application in US patent published No. 20030187434 A1 discloses a vertebral fixation device comprising a fixation seat, a fixation block, and a fastening bolt. The fixation seat is provided with a receiving slot for receiving a vertebral fixation rod. The fixation block is engaged with the fixation seat such that the vertebral fixation rod is pressed by the fixation block, and that two retaining edges of the fixation block are retained in two retaining recesses of the fixation seat. The fastening bolt is engaged with a threaded through hole of said fixation block such that one end of the fastening bolt presses against the vertebral fixation rod. Although this design improves the conventional backbone-fixing devices, there are at least the following defects which call for further improvements: 1) the contact of the spherical head fastening screw and the fixation rod is restricted to a thin line; and 2) the fixation block has to be fabricated precisely to well fasten the fixation rod in the receiving slot of the fixation seats. For this reason, the fixation rod is susceptible to unintentional displacement in a spinal surgery in progress. Such a displacement of the back-retrieving rod often affects adversely the outcome of the spinal surgery. US patent No. 6,565,565 B1 discloses a device for securing spinal rods, which is similar to that described in the US patent published No. 20030187434 A1 with the common drawbacks.

The document WO 01/52758 A discloses a vertebral retrieval device which comprises a retrieval rod, a fixation seat, a fastening screw, a C-shaped ring and a fixation assembly. The fixation seat is provided with a receiving slot for receiving the retrieval rod, being provided on two opposite side walls with a retaining recess, and in a bottom with a through hole, the fastening screw is provided with a head made integrally therewith, wherein the head is rotatably received in said fixation seat when the fastening screw is inserted into the through hole of the fixation seat, the C-shaped ring being able to be elastically deformed, received in the fixation seat and retained in the fixation seat by an expansion force resulting from said deformation. The fixation assembly of the vertebral retrieval device comprises a rotary fixation element provided with a through hole, and two retaining edges opposite to each other, a pressing element having a projection at one side and a groove at another side, the rotary fixation element being engaged with said fixation seat such that the two retaining edges are retained in the retaining recesses of the fixation seat.

### SUMMARY OF THE INVENTION

A primary objective of the present invention is to provide a rotary device for fixing spinal column under treatment without the drawbacks of the prior art.

A vertebral retrieval device constructed according to the present invention comprises:
a retrieval rod which is used to retrieve spinal segments under treatment;
a fixation seat provided with a receiving slot for receiving the retrieval rod, said receiving slot being provided on two opposite side walls with a retaining recess, and in a bottom with a through hole;
a fastening screw is provided with a spherical head made integrally therewith, wherein said spherical head is rotatably received in said fixation seat when said fastening screw is inserted into said through hole of said fixation seat;
a C-shaped ring provided with a concave down surface on one side, said C-shaped ring being able to be elastically deformed, received in said fixation seat and retained in said fixation seat by an expansion force resulting from said deformation, while said concave down surface resting on the spherical head of said fastening screw and another side of said C-shaped ring being adapted to receive said retrieval rod;
a fixation assembly comprising a rotary fixation element provided with a threaded through hole, and two retaining edges opposite to each other; a pressing element having a projection at one side and a groove at another side; and a pressing screw having a round hole adapted to receive said projection of said pressing element, wherein said rotary fixation element is engaged with said fixation seat such that the two retaining edges are retained in said retaining recesses of said fixation seat, said pressing screw is threadably engaged in said threaded through hole of said rotary fixation element, and the pressing element is able to be pressed against said retrieval rod by threading said pressing screw, when the groove of said pressing element rests on said retrieval rod and said projection of said pressing element is received in said round hole of said pressing screw.

Preferably, said retaining recesses of said fixation seat are located in inner sides of the two side walls of said receiving slot; wherein said retaining edges of said rotary fixation element are located in outer edges of said rotary fixation element. More preferably, said retaining edges of said rotary fixation element are provided with an inclined plane having a higher outer side and a lower inner side; wherein said retaining recesses of said fixation seat are provided with an inclined plane having a higher outer side and a lower inner side, wherein said rotary fixation element is engaged with said fixation seat such that said inclined planes of said rotary fixation element and said fixation seat are in contact with each other, and thus the two side walls of said receiving slot of said fixation seat are further prevented from departing from each other.

Preferably, said C-shaped ring is provided with a groove on said another side thereof, and said groove of said C-shaped ring is adapted to receive said retrieval rod;

Preferably, a tool hole is provided in said pressing screw for facilitating the threadable engagement with said rotary fixation element.

Said C-shaped ring of the present invention is elastically retained in the fixation seat and is pressed against the spherical head of the fastening screw, creating more friction between the C-shaped ring and the fixation seat/the spherical head of the fastening screw, so that the fastening thereof is improved. The groove of the C-shaped ring further enhances the friction and the fastening effect.

The pressing screw of the present invention can be threaded to press the pressing element against the fixation rod, which presses the C-shaped ring, which presses the spherical head of the fastening screw, which presses the fastening seat, so that the retrieval rod is gripped firmly in the receiving slot of the fixation seat, and thus it is secured to the fastening screw which has be fastened to a pedicle of a vertebra in advance.

The features and the advantages of the present invention will be more readily understood upon a thoughtful deliberation of the following detailed description of the present invention with reference to the accompanying drawings.

### BRIEF DESCRIPTION OF THE DRAWINGS

FIG. 1 shows an exploded view of a first preferred embodiment of the present invention.
FIG. 2 shows a perspective view of the first preferred embodiment of the present invention shown in FIG. 1, when the fixation assembly 60 is assembled.
FIG. 3 shows a perspective view of the first preferred embodiment of the present invention shown in FIG. 1 at work.
FIG. 4 shows a sectional view of the first preferred embodiment of the present invention shown in FIG. 3.

### DETAILED DESCRIPTION OF THE PREFERRED EMBODIMENT

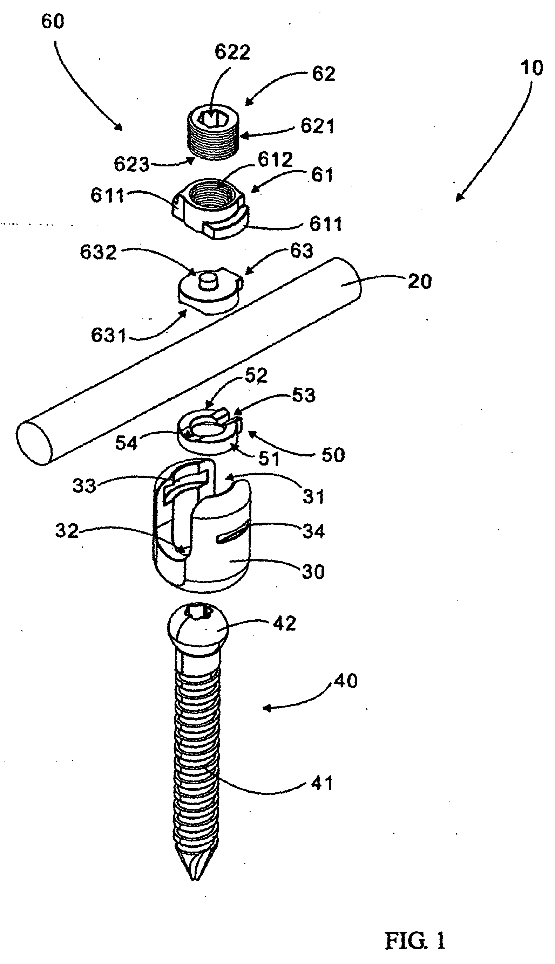

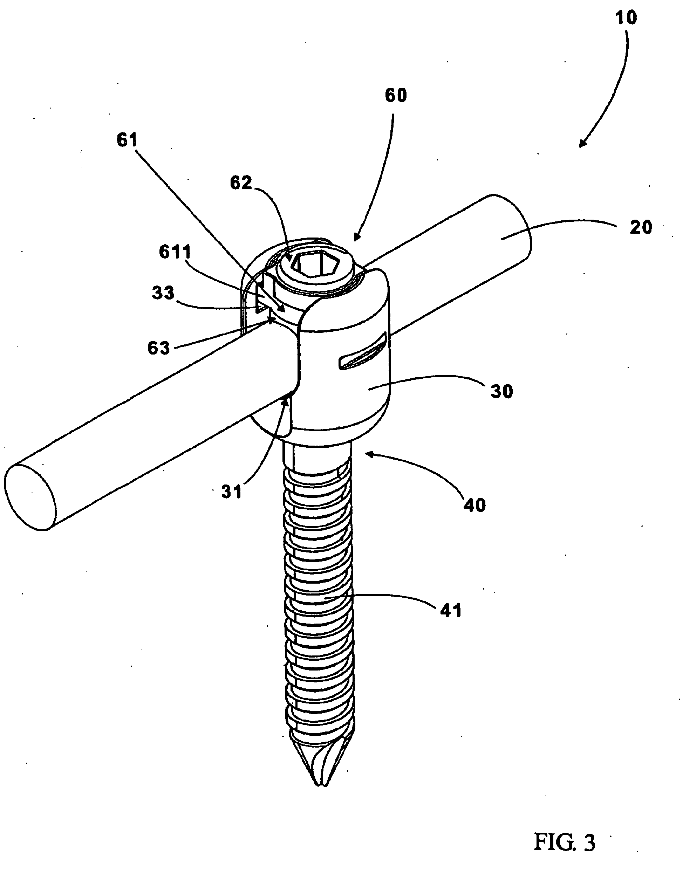

As shown in FIGS. 1-4, a vertebral retrieval device 10 of the present invention comprises a retrieval rod 20, a fixation seat 30, a fastening screw 40, a C-shaped ring 50, and a fixation assembly 60.

The fixation seat 30 is provided with a U-shaped receiving slot 31 which has two retaining recesses 33 on the two inner walls, two clamping slots 34 on the outer walls, and a through hole 32 in the bottom.

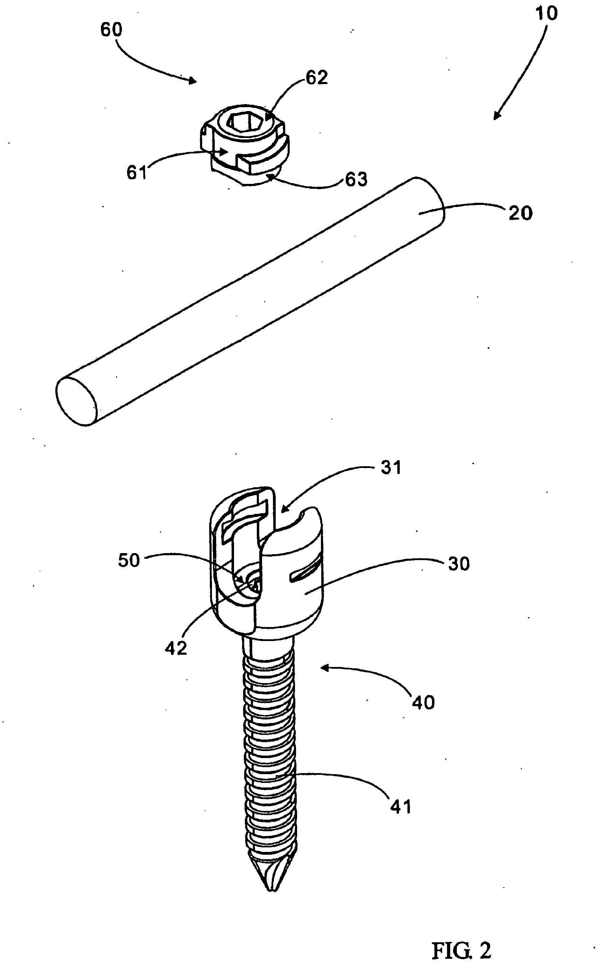

The fixation assembly 60 has a rotary fixation element 61, a pressing screw 62 and a pressing element 63, which can be assemble before using, as shown in FIG. 2. The rotary fixation element 61 is provided with a threaded through hole 612 at the axial center, and two opposite retaining edges 611 protruding radically from the outer surface. The pressing element 63 has a cylindrical projection 632 at one side and a groove 631 at another side. The pressing screw 62 has a round hole 623 for receiving said projection 632 of said pressing element, and is threadably engaged in the threaded through hole 612 of the rotary fixation element 61.

The fastening screw is provided with a shank 41 and a spherical head 42 made integrally therewith, wherein said spherical head 42 is rotatably received in said fixation seat 30 when said shank 41 is protruding from said through hole 32 of said fixation seat. The shank 41 of the fastening screw 41 is then fastened to a pedicle of a vertebra under treatment (not shown in the drawings).

The C-shaped ring 50 is provided with a concave down surface 51 on one side, and a groove 54 adapted to receive said retrieval rod 20 on another side 52 thereof. Said C-shaped ring 50 can be elastically deformed due to an opening 53, received in said fixation seat 30 and retained in said fixation seat by an expansion force resulting from said deformation, while said concave down surface 51 resting on the spherical head 42 of said fastening screw 40.

The retrieval rod 20 is received in the receiving slot 31 of the fixation seat 30 and on the groove 54 of the C-shaped ring 50.

The fixation assembly 60 is rotated and connected to the fixation seat 30, wherein the rotary fixation element 61 is engaged with said fixation seat 30 such that the two retaining edges 611 are retained in said retaining recesses 33 of said fixation seat 30. The pressing element 63 is able to be pressed against said retrieval rod 20 by threading said pressing screw 62, wherein the groove 631 of said pressing element rests on said retrieval rod 62 and said projection 632 of said pressing element is received in said round hole 623 of said pressing screw. The pressing screw 62 is provided with a tool hole 622 for facilitating the threadable engagement with said rotary fixation element 61 and the pressing.

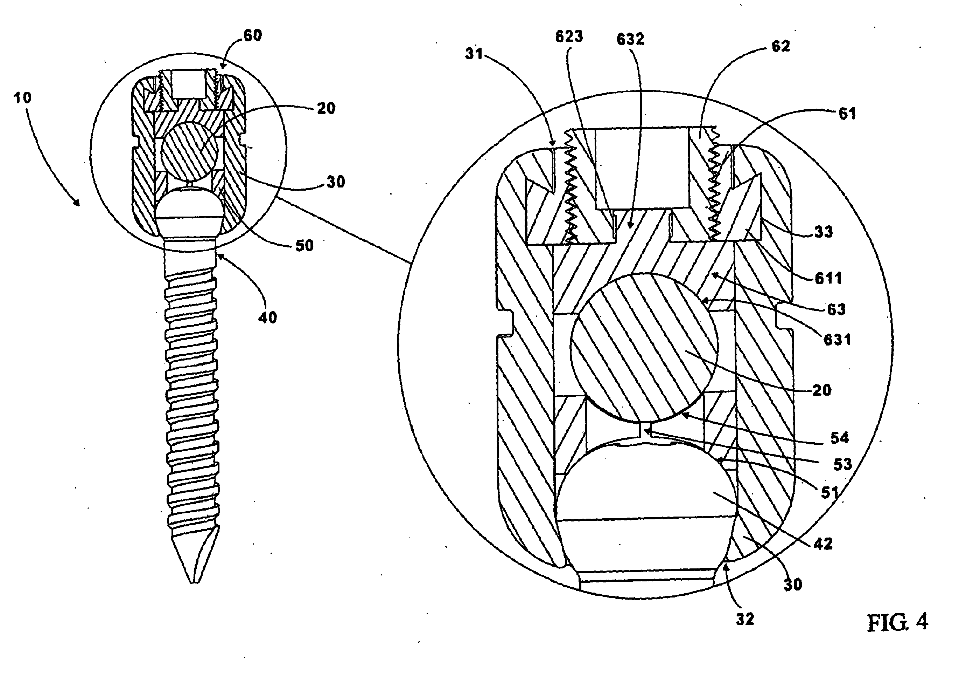

As shown in FIG.4, the retaining edges 611 of said rotary fixation element are provided with an inclined plane having a higher outer side and a lower inner side; and the retaining recesses 33 of said fixation seat 30 are provided with an inclined plane having a higher outer side and a lower inner side, wherein said rotary fixation element 61 is engaged with said fixation seat 30 such that said inclined planes of said rotary fixation element 61 and said fixation seat 30 are in contact with each other, and thus the two side walls of said receiving slot 31 of said fixation seat are held by the rotary fixation element 61.

## Claims

1. A vertebral retrieval device (10) comprising:
a retrieval rod (20) which is used to retrieve spinal segments under treatment;
a fixation seat (30) provided with a receiving slot (31) for receiving the retrieval rod (20), said receiving slot (31) being provided on two opposite side walls with a retaining recess (33), and in a bottom with a through hole (32);
a fastening screw (40) is provided with a spherical head (42) made integrally therewith, wherein said spherical head (42) is rotatably received in said fixation seat (30) when said fastening screw (40) is inserted into said through hole (32) of said fixation seat (30);
a C-shaped ring (50) provided with a concave down surface (51) on one side, said C-shaped ring (50) being able to be elastically deformed, received in said fixation seat (30) and retained in said fixation seat (30) by an expansion force resulting from said deformation, while said concave down surface (51) resting on the spherical head (42) of said fastening screw (40) and another side of said C-shaped ring (50) being adapted to receive said retrieval rod (20);
a fixation assembly (60) comprising a rotary fixation element (61) provided with a threaded through hole (612), and two retaining edges (611) opposite to each other; a pressing element (63) having a projection (632) at one side and a groove (631) at another side; and a pressing screw (62) having a round hole (623) adapted to receive said projection (632) of said pressing element (63), wherein said rotary fixation element (61) is engaged with said fixation seat such that the two retaining edges (611) are retained in said retaining recesses (33) of said fixation seat (30), said pressing screw (62) is threadably engaged in said threaded through hole (612) of said rotary fixation element (61), and the pressing element (63) is able to be pressed against said retrieval rod (20) by threading said pressing screw (62), when the groove (631) of said pressing element (63) tests on said retrieval rod (20) and said projection (632) of said pressing element (63) is received in said round hole (623) of said pressing screw (62).

2. The device as defined in claim 1, wherein said retaining recesses (33) of said fixation seat (30) are located in inner sides of the two side walls of said receiving slot (31), wherein said retaining edges (611) of said rotary fixation element (61) are located in outer edges of said rotary fixation element (61).

3. The device as defined in claim 2, wherein said retaining edges (611) of said rotary fixation element (61) are provided with an inclined plane having a higher outer side and a lower inner side; wherein said retaining recesses (33) of said fixation seat (30) are provided with an inclined plane having a higher outer side and a lower inner side, wherein said rotary fixation element (61) is engaged with said fixation seat (30) such that said inclined planes of said rotary fixation element (61) and said fixation seat (30) are in contact with each other, and thus the two side walls of said receiving slot (31) of said fixation seat (30) are prevented from departing from each other.

4. The device as defined in claim 1, wherein said C-shaped ring (50) is provided with a groove (54) on said another side thereof, and said groove (54) of said C-shaped ring (50) is adapted to receive said retrieval rod (20);

5. The device as defined in claim 1, wherein a tool hole (622) is provided in said pressing screw (62) for facilitating the threadable engagement with said rotary fixation element (61).

## Patentansprüche

1. Wirbel-Wiederherstellungsvorrichtung (10), die umfasst:
eine Wiederherstellungsstange (20), die eingesetzt wird, um in Behandlung befindliche Wirbelsäulensegmente wiederherzustellen,
eine Fixieraufnahme (30), die mit einem Aufnahmeschlitz (31) zum Aufnehmen der Wiederherstellungsstange (20) versehen ist, wobei der Aufnahmeschlitz (31) an zwei einander gegenüberliegenden Seitenwänden mit einer Halteaussparung (33) und an einer Unterseite mit einem Durchgangsloch (32) versehen ist;
eine Befestigungsschraube (40), die mit einem kugelförmigen Kopf (42) versehen ist, der integral damit ausgebildet ist, wobei der kugelförmige Kopf (42) drehbar in der Fixieraufnahme (30) aufgenommen ist, wenn die Befestigungsschraube (40) in das Durchgangsloch (32) der Fixieraufnahme (30) eingeführt wird;
einen C-förmigen Ring (50), der mit einer konkaven unteren Fläche (51) an einer Seite versehen ist, wobei der C-förmige Ring (50) elastisch verformt werden kann, in der Fixieraufnahme (30) aufgenommen werden kann und durch eine aus der Verformung resultierende Ausdehnungskraft in der Fixieraufnahme (30) gehalten werden kann, wobei die konkave untere Fläche (51) auf dem kugelförmigen Kopf (42) der Befestigungsschraube (40) aufliegt und eine andere Seite des C-förmigen Rings (50) so eingerichtet ist, dass sie die Wiederherstellungsstange (20) aufnimmt;
eine Fixierbaugruppe (60), die ein drehbares Fixierelement (61), das mit einem mit Gewinde versehenen Durchgangsloch (612) und zwei einander gegenüberliegenden Haltekanten (611) versehen ist, ein Presselement (63), das einen Vorsprung (632) an einer Seite und eine Nut (631) an der anderen Seite hat, und eine Pressschraube (62) umfasst, die ein Rundloch (623) hat, das so eingerichtet ist, dass es den Vorsprung (632) des Presselementes (63) aufnimmt, wobei das drehbare Fixierelement (61) mit der Fixieraufnahme so in Eingriff ist, dass die zwei Haltekanten (611) in der Haltevertiefung (33) der Fixieraufnahme (30) gehalten werden, die Pressschraube (62) in Gewindeeingriff mit dem mit Gewinde versehenen Durchgangsloch (612) des drehbaren Fixierelementes (61) ist, und das Presselement (63) an die Wiederherstellungsstange (20) gepresst werden kann, indem die Pressschraube (62) geschraubt wird, wenn die Nut (633) des Presselementes (63) auf der Wiederherstellungsstange (20) aufliegt, und der Vorsprung (632) des Presselementes (63) in dem Rundloch (623) der Pressschraube (62) aufgenommen ist.

2. Vorrichtung nach Anspruch 1, wobei die Haltevertiefungen (33) der Fixieraufnahme (30) an Innenseiten der zwei Seitenwände des Aufnahmeschlitzes (31) angeordnet sind, wobei die Haltekanten (611) des drehbaren Fixierelementes (61) an Außenkanten des drehbaren Fixierelementes (61) angeordnet sind.

3. Vorrichtung nach Anspruch 2, wobei die Haltekanten (611) des drehbaren Fixierelementes (61) mit einer geneigten Ebene versehen ist, die eine höhere Außenseite und eine niedrigere Innenseite hat, die Haltevertiefungen (33) der Fixieraufnahme (30) mit einer geneigten Ebene versehen sind, die eine höhere Außenseite und eine niedrigere Innenseite hat, das drehbare Fixierelement (61) mit der Fixieraufnahme (30) so in Eingriff ist, dass die geneigten Ebenen des drehbaren Fixierelementes (61) und die Fixieraufnahme (30) in Kontakt miteinander sind und so die zwei Seitenwände des Aufnahmeschlitzes (31) der Fixieraufnahme (30) daran gehindert werden, sich voneinander zu lösen.

4. Vorrichtung nach Anspruch 1, wobei der C-förmige Ring (50) mit einer Nut (54) an seiner anderen Seite versehen ist und die Nut (54) des C-förmigen Rings (50) so eingerichtet ist, dass sie die Wiederherstellungsstange (20) aufnimmt.

5. Vorrichtung nach Anspruch 1, wobei ein Werkzeugloch (622) in der Pressschraube (62) vorhanden ist, um den Gewindeeingriff mit dem drehbaren Fixierelement (61) zu erleichtern.

## Revendications

1. Dispositif de redressement vertébral (10) comprenant :
une tige de redressement (20) utilisée pour redresser des segments de colonne vertébrale sous traitement ;
un siège de fixation (30) muni d'une fente de réception (31) destinée à recevoir la tige de redressement (20), ladite fente de réception (31) étant pourvue d'un évidement de retenue (33) sur deux parois latérales opposées et d'un trou traversant (32) dans une partie formant fond ;
une vis de fixation (40) munie d'une tête sphérique (42) réalisée solidairement avec elle, ladite tête sphérique (42) étant reçue de façon à pouvoir tourner dans ledit siège de fixation (30) lorsque ladite vis de fixation (40) est insérée dans ledit trou traversant (32) dudit siège de fixation (30) ;
un anneau en forme de C (50) dont un côté est muni d'une surface inclinée concave (51), ledit anneau en forme de C (50) pouvant être déformé élastiquement, et étant reçu dans ledit siège de fixation (30) et maintenu dans celui-ci par une force d'expansion résultant de ladite déformation, ladite surface inclinée concave (51) reposant sur la tête sphérique (42) de ladite vis de fixation (40), et un autre côté dudit anneau en forme de C (50) étant adapté pour recevoir ladite tige de redressement (20) ;
un ensemble de fixation (60) comprenant un élément de fixation rotatif (61) muni d'un trou traversant fileté (612), et deux bords de retenue (611) opposés l'un à l'autre ; un élément de pression (63) comportant une saillie (632) sur un côté et une gorge (631) sur un autre côté ; et une vis de pression (62) comportant un trou rond (623) adapté pour recevoir ladite saillie (632) dudit élément de pression (63), ledit élément de fixation rotatif (61) étant en prise avec ledit siège de fixation de telle sorte que les deux bords de retenue (611) soient retenus dans lesdits évidements de retenue (33) dudit siège de fixation (30), ladite vis de pression (62) étant retenue par vissage dans ledit trou traversant fileté (612) dudit élément de fixation rotatif (61), et l'élément de pression (63) pouvant être pressé contre ladite tige de redressement (20) par vissage de ladite vis de pression (62) lorsque la gorge (631) dudit élément de pression (63) repose sur ladite tige de redressement (20) et ladite saillie (632) dudit élément de pression (63) est reçue dans ledit trou rond (623) de ladite vis de pression (62).

2. Dispositif selon la revendication 1, dans lequel lesdits évidements de retenue (33) dudit siège de fixation (30) sont situés dans les faces intérieures des deux parois latérales de ladite fente de réception (31), lesdits bords de retenue (611) dudit élément de fixation rotatif (61) étant situés dans des bords extérieurs dudit élément de fixation rotatif (61).

3. Dispositif selon la revendication 2, dans lequel lesdits bords de retenue (611) dudit élément de fixation rotatif (61) sont munis d'un plan incliné comportant un côté extérieur plus haut et un côté intérieur plus bas ; dans lequel lesdits évidements de retenue (33) dudit siège de fixation (30) sont munis d'un plan incliné comportant un côté extérieur plus haut et un côté intérieur plus bas, dans lequel ledit élément de fixation rotatif (61) est en prise avec ledit siège de fixation (30) de telle sorte que lesdits plans inclinés dudit élément de fixation rotatif (61) et dudit siège de fixation (30) soient en contact l'un avec l'autre et qu'ainsi les deux parois latérales de ladite fente de réception (31) dudit siège de fixation (30) ne puissent s'éloigner l'une de l'autre.

4. Dispositif selon la revendication 1, dans lequel ledit anneau en forme de C (50) est muni d'une gorge (54) sur son autre côté, ladite gorge (54) dudit anneau en forme de C (50) étant adaptée pour recevoir ladite tige de redressement (20) ;

5. Dispositif selon la revendication 1, dans lequel un trou d'outil (622) est prévu dans ladite vis de pression (62) pour faciliter la mise en prise, par vissage, de celle-ci avec ledit élément de fixation rotatif (61).
